# NEUE EUROPÄISCHE PATENTSCHRIFT

(11) **EP 2 470 496 B2**
(45) Veröffentlichungstag und Bekanntmachung des Hinweises auf die Entscheidung über den Einspruch: **27.03.2024**
(45) Hinweis auf die Patenterteilung: 06.01.2016
(21) Anmeldenummer: 10747834.9
(22) Anmeldetag: 25.08.2010
(51) Int. Cl.: C07C 227/42, C07C 229/16, C07C 229/76, C11D 3/33, C11D 7/32

(54) **VERFAHREN ZUR HERSTELLUNG EINES KRISTALLINEN FESTSTOFFES AUS GLYCIN-N, N-DIESSIGSÄURE-DERIVATEN**
METHOD FOR PRODUCING A CRYSTALLINE SOLID FROM GLYCINE-N, N-DIACETIC ACID DERIVATIVES
PROCÉDÉ DE PRODUCTION D'UN SOLIDE CRISTALLIN À PARTIR DE DÉRIVÉS DE L'ACIDE GLYCINE-N, N-DIACÉTIQUE

(30) Priorität: 26.08.2009 DE 102009038951
(43) Veröffentlichungstag der Anmeldung: 04.07.2012
(73) Patentinhaber: BASF SE, 67056 Ludwigshafen am Rhein (DE)
(72) Erfinder: BARANYAI, Andreas, 79423 Heitersheim (DE)
(74) Vertreter: BASF IP Association
(86) Internationale Anmeldenummer: PCT/EP2010/005219
(87) Internationale Veröffentlichungsnummer: WO 2011/023382

(56) Entgegenhaltungen:
- EP-A1- 2 161 075
- WO-A1-2009/103822
- WO-A1-2009/103822
- DE-A1- 19 649 681
- Trilon M brochure of 2007,
- ULLMANN: Encyclopedia - Fertilizers Granulation, 2009, pages 253-272,
- H. GROENEWOLD: "Die jeweiligen stärken nutzen", Chemie Technik, March 2006 (2006-03), pages 44-46,
- , Retrieved from the Internet: URL:http://www.omega.com/temperature/z/pdf /z103.pdf

## Beschreibung

Komplexbildner für Erdalkali- und Schwermetallionen, wie sie beispielsweise in Wasch- und Reinigungsmitteln eingesetzt werden, werden üblicherweise in wässriger Lösung synthetisiert. Für bestimmte Anwendungsfälle werden sie in fester Form benötigt.

Übliche Verfahren zur Herstellung von Feststoffen aus Lösungen sind insbesondere Kristallisations- und Sprühtrocknungsverfahren. Bekannt ist, dass kristalliner Feststoff, wie er beispielsweise bei Verdampfungs- oder Kühlkristallisationsverfahren anfällt, Kristallwasser enthalten kann und unter Umgebungsbedingungen meist weniger hygroskopisch und lagerstabiler als amorpher Feststoff ist. Durch Sprühtrocknungsverfahren (z. B. im Sprühturm oder im Sprühwirbelbett) dagegen wird der Feststoff amorph erhalten. In dieser Form ist der Feststoff oft stark hygroskopisch und bei offener Lagerung unter Umgebungsbedingungen verliert er innerhalb kurzer Zeit die Rieselfähigkeit. Deshalb werden in der Literatur Maßnahmen beschrieben, um die Lagerstabilität von Sprühpulver zu erhöhen, z. B. die Kompaktierung oder Nachbehandlung von Buildern für Waschmittel mit Benzoesäure in der US 3,932,316.

Glycin-N,N-diessigsäure-Derivate als Komplexbildner für Erdalkali- und Schwermetallionen in verschiedensten technischen Anwendungsgebieten sind aus der WO 94/29421 bekannt. Diese Glycin-N,N-diessigsäure-Derivate, z. B. Methylglycin-N,N-diessigsäure (MGDA) in Form des Trinatriumsalzes, kristallieren stark gehemmt, so dass übliche Kristallisationsverfahren nicht möglich oder nicht wirtschaftlich sind. Die Nachbehandlung von amorphem Sprühpulver dieser Verbindungen mit Additiven, z. B. Benzoesäure gemäss der US 3,932,316, ist für einige Anwendungen nicht erwünscht und kann auch nur in begrenztem Umfang die Lagerstabilität verbessern. Die Stabilität eines kristallinen Feststoffes wird nicht erreicht.

WO 2009/103822 offenbart ein Verfahren zur Herstellung von Methylglycindiessigsäuregranulaten durch Versprühen einer Suspension von MGDA bei einer Gaseintrittstemperatur von 120°C oder weniger. Als Keim wird MGDA-Pulver vorgelegt.

EP 2 161 075 A1 offenbart die Sprühtrocknung von bestimmten Übergangsmetallsalzen von Glycin.

Aufgabe der vorliegenden Erfindung war es daher, einen praktisch nichthygroskopischen, stabilen, vorzugsweise kristallinen Feststoff aus im wesentlichen Glycin-N,N-diessigsäure-Derivaten bereitzustellen, der weitgehend frei von Additiven ist.

Die vorliegende Erfindung betrifft ein Verfahren zur Herstellung eines kristallinen Feststoffes, welcher z.B. für eine Verarbeitung und Anwendung eine hinreichend geringe Hygroskopizität aufweist und im wesentlichen Methylglycin-N,N-diessigsäure-Derivate der allgemeinen Formel I umfasst 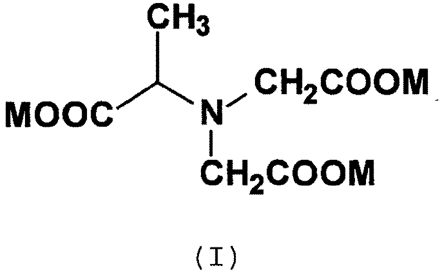 in der jedes M unabhängig voneinander Wasserstoff, Alkalimetall, Ammonium oder substituiertes Ammonium (z.B. organische Aminsalze) in den entsprechenden stöchiometrischen Mengen bedeutet, welches dadurch gekennzeichnet ist, dass mindestens eine kristalline Verbindung der Formel I als Keim vorgelegt wird, und eine Sprühgranulation (vorzugsweise in einem Granulator) mit einer wässrigen Lösung von einer Verbindung der Formel I durchgeführt wird.

Insbesondere bevorzugt sind Verbindungen der Formel I, wie sie in DE 196 49 681 beschrieben sind.

In an sich üblicher Weise werden bei einer Sprühgranulation kleine bis kleinste Tröpfchen im Bereich von 50µm einer durch Düsen versprühten Flüssigkeit in einer Reaktionskammer durch direkte Wärmeübertragung in einem warmen oder heißen Luftstrom zu Partikeln getrocknet. Aus wässrigen Lösungen, Emulsionen oder Dispersionen werden z.B. zuerst durch Trocknung dieser versprühten Tröpfchen in der Reaktionskammer kleinste Partikel (Keime) erzeugt (Alternativ können diese Keime auch vorgelegt werden). Dabei werden diese Keime in einer Wirbelschicht im Schwebezustand (Fluidisierung) gehalten und bilden die Oberfläche zu einer schichtweisen Adsorption und Trocknung weiterer verdüster Tröpfchen. Die so erzeugten Partikel können durch einen klassierenden Austrag flexibel - z.B. mit frei einstellbaren Partikelgrößen - ohne eine Unterbrechung des Trocknungsvorganges aus dem Trocknungsraum kontinuierlich entfernt werden. Zum Verfahren der Sprühgranulation siehe auch H. Uhlemann, L. Mörl, "Wirbelschicht - Sprühgranulation" Springer - Verlag 2000 (ISBN 3-540-66985-X).

Das Verfahren der vorliegenden Erfindung ist dadurch gekennzeichnet, dass mindestens eine kristalline Verbindung der Formel I als Keime vorgelegt wird und dann in an sich üblicher Weise eine Sprühgranulation mit mindestens einer Verbindung der Formel I in Lösung (insbesondere in wässriger Lösung, z.B. ca. 40%ig) durchgeführt wird.

Vorzugsweise wird eine Sprühgranulation mit folgenden Parametern durchgeführt:
Bevorzugte Zulufttemperatur: 90-160°C, bevorzugte Ablufttemperatur: 40-110°C, bevorzugte Produkttemperatur: 40-110°C, bevorzugte Temperatur der Sprühluft: 70-110°C, bevorzugter Druck der Sprühluft: 1-6 bar, bevorzugte Temperatur der Sprühlösung: 50-95°C.

Bei dem erfindungsgemäßen Verfahren wird beispielsweise flüssiger Rohstoff (z.B. eine 40%ige wässrige Lösung einer Verbindung der Formel I) auf die im heißen Luftstrom wirbelnden Kristallkeime (von Verbindungen der Formel I) aufgesprüht, der dadurch trocknet und die Keime zum Wachsen bringt. Dieser Prozess wird vorzugsweise kontinuierlich betrieben, wobei vorzugsweise kontinuierlich ein Teil des Produktes aus dem Granulator abgezogen wird, der dann mit einem zusätzlichen Temperschritt (Wärmebehandlungsschritt) behandelt wird. Hierdurch wird die Hygroskopizität des Produktes erniedrigt, vorzugsweise durch eine Erhöhung des kristallinen Anteils im Produkt. Das so behandelte Produkt stellt das Endprodukt dar, wobei wiederum vorzugsweise ein Teil gemahlen wird und als neue Keime wieder in den Granulator eingebracht wird.

Vorzugsweise wird das Produkt mit folgendem Temperaturprofil Wärme-nachbehandelt (getempert): Beginnend mit einer Betttemperatur von 50-90°C wird innerhalb von ca. einer Stunde die Betttemperatur auf 90-130°C erhöht und anschließend für ca. 60 Minuten bei dieser Temperatur gehalten.

Der Granulator ist vorzugsweise ein Fließbett Sprühgranulator, der beispielsweise mit einem Zyklon und / oder einem Filter ausgestattet ist.

Bei dem Verfahren der vorliegenden Erfindung muss man vorzugsweise nur ganz zu Beginn kristalline Ware vorlegen und erhält dann durch eine Sprühgranulation (durch die man ohne Vorlage von kristalliner Ware z.B. nur weit mehr amorphes Granulat erhalten würde) und die nachfolgende Temperung (Wärmebehandlung) dauerhaft ein Produkt mit höherem kristallinen Anteil (und dadurch wesentlich weniger Hygroskopizität).

Der Ausdruck "kristallin" bezieht sich vorzugsweise auf einen kristallinen Anteil von mindestens Gew.-60%.

Als nichthygroskopisch bzw. hinreichend gering hygroskopisch wird hier ein Feststoff bezeichnet, der bei der offenen Lagerung unter normalen Umgebungsbedingungen, z. B. 25°C und einer relativen Luftfeuchte von 76%, über einen Zeitraum von mindestens einem Tag, vorzugsweise einer Woche seine Konsistenz als (vorzugsweise rieselfähiges) Pulver oder Granulat bewahrt.

Der erfindungsgemäß hergestellte kristalline Feststoff umfasst Methylglycin-N,N-diessigsäure, wobei geringe Mengen von Ausgangs- und/oder Nebenprodukten aus der Herstellung der Methylglycin-N,N-diessigsäure zusätzlich vorliegen können. Übliche Reinheiten für die Verbindungen I liegen, je nach angewandtem Syntheseverfahren, bei 70 bis 99,9 Gew.-%, insbesondere bei 80 bis 99,5 Gew.-%, jeweils bezogen auf den Feststoffgehalt.

Die kristalline Ausgangssubstanz kann beispielsweise durch das in DE 196 49 681 beschriebene Verfahren hergestellt werden. Erfindungsgemäß werden als Verbindung I Methylglycin-N,N-diessigsäure (MGDA) und ihre Salze eingesetzt. Bevorzugt werden z.B. ihre Alkalimetall-, Ammonium- und substituierten Ammoniumsalze eingesetzt.

Als derartige Salze eignen sich vor allem die Natrium-, Kalium- und Ammoniumsalze, insbesondere das Trinatrium-, Trikalium- und Triammoniumsalz sowie organische Triaminsalze mit einem tertiären Stickstoffatom.

Als den organischen Aminsalzen zugrundeliegende Basen kommen insbesondere tertiäre Amine, wie Trialkylamine mit 1 bis 4 C-Atomen im Alkyl, wie Trimethyl- und Triethylamin, und Trialkanolamine mit 2 oder 3 C-Atomen im Alkanolrest, bevorzugt Triethanolamin, Tri-n-propanolamin oder Triisopropanolamin, in Betracht.

Als Erdalkalimetallsalze werden insbesondere die Calcium- und Magnesiumsalze eingesetzt.

Der erfindungsgemäß hergestellte kristalline Feststoff eignet sich in besonderem Maße als Komponente für feste Wasch- und Reinigungsmittelformulierungen. Daher sind hier auch feste Wasch- und Reinigungsmittelformulierungen, die das erfindungsgemäß hergestellten kristalline MGDA mit hinreichend geringer Hygroskopizität als Komplexbildner für Erdalkali- und Schwermetallionen in den hierfür üblichen Mengen neben anderen üblichen Bestandteilen solcher Formulierungen enthalten, erwähn.

Zusammensetzungen und übliche Bestandteile derartiger fester Wasch- und Reinigungsmittelformulierungen sind dem Fachmann bekannt und brauchen daher hier nicht näher erläutert zu werden.

Das nachfolgende Beispiel soll die Erfindung näher erläutern. Methylglycin-N,N-diessigsäure (α-Alanin-N,N-diessigsäure, MGDA) wird als Trinatriumsalz eingesetzt.

### Beispiel

Der folgende Prozess wurde in einem Fließbett Sprühgranulator durchgeführt, der mit einem Zyklon, einem Filter und einem Gaswäscher ausgestattet ist.

Das Ausgangsmaterial MGDA Trinatriumsalz (Trilon^{®} M flüssig der BASF AG) wurde unter kontinuierlichem und intensiven Durchmischen auf 90 °C erhitzt und für die Granulation bei dieser Temperatur verwendet. Bei den folgenden Bedingungen wurde ein stabiler Granulationsprozess erreicht:

| Prozessparameter: | |
|---|---|
| Zulufttemperatur | 125 °C |
| Ablufttemperatur | 65 °C |
| Produkttemperatur | 65 - 70 °C |
| Ansaugluftvolumen | 1300 m³/h |
| Temperatur der Sprühluft | 90 °C |
| Druck der Sprühluft | 3 bar |
| Temperatur der Sprühlösung | 80 °C |

Um die Kristallinität zu erhöhen, wurde das hergestellte Produkt mit einem Temperaturprofil nachbehandelt wobei mit einer Betttemperatur von 70 °C begonnen wurde, diese dann innerhalb etwa einer Stunde auf etwa 110 - 120 °C erhöht wurde und anschließend für etwa 60 Minuten bei dieser Temperatur gehalten wurde.

Sieben bei 1000 Mikron und Wiederverwendung des gemahlenen Grobguts als Kristallisationskeime für das Granulationsverfahren führte zu einem stabilen Prozess mit einem Ertrag von etwa 20 kg Granulat pro Stunde in der gewünschten Qualität. Die Einspeisung von zerkleinertem Material war vorteilhaft für den Prozess, um die Höhe des Betts zu bewahren und um das Produkt in einer kristallinen Form zu erhalten.

## Patentansprüche

1. Verfahren zur Herstellung eines kristallinen Feststoffes, welcher - bezogen auf den Feststoffgehalt - 70 bis 99,9 Gew.-% Methylglycin-N,N-diessigsäure-Derivate der allgemeinen Formel I umfasst 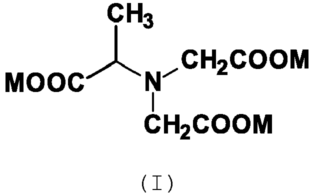
in der jedes M unabhängig voneinander Wasserstoff, Alkalimetall, Ammonium oder substituiertes Ammonium in den entsprechenden stöchiometrischen Mengen bedeutet,
wobei der Feststoff bei der offenen Lagerung unter 25°C und einer relativen Luftfeuchte von 76% über einen Zeitraum von mindestens einem Tag seine Konsistenz als Granulat bewahrt,
**dadurch gekennzeichnet, dass** mindestens eine kristalline Verbindung der Formel I vorgelegt wird und eine Sprühgranulation mit einer wässrigen Lösung von einer Verbindung der Formel I durchgeführt wird.

2. Verfahren nach Anspruch 1, **dadurch gekennzeichnet, dass** ein Teil des Produktes aus dem Granulator abgezogen wird, gemahlen wird und als neue Keime in den Granulator eingeführt wird.

3. Verfahren nach einem der Ansprüche 1 bis 2, wobei das Produkt Wärme-nachbehandelt wird.

## Claims

1. A process for preparing a crystalline solid comprising - based on the solids content - 70% to 99.9% by weight of methylglycine-N,N-diacetic acid derivatives of the general formula I 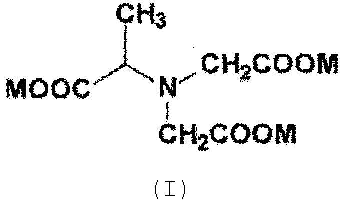
in which each M independently of any other is hydrogen, alkali metal, ammonium or substituted ammonium in the corresponding stoichiometric amounts,
where, on storage in the open at 25°C and a relative humidity of 76%, the solid preserves its consistency as granules over a period of at least one day,
wherein at least one crystalline compound of the formula I is introduced and a spray granulation is carried out with an aqueous solution of a compound of the formula I.

2. The process according to claim 1, wherein part of the product is removed from the granulator, ground, and introduced as new seeds into the granulator.

3. The process according to either of claims 1 and 2, the product being heat-aftertreated.

## Revendications

1. Procédé de fabrication d'un solide cristallin qui comprend, par rapport à la teneur en solides, 70 à 99,9 % en poids de dérivés de l'acide méthylglycine-N,N-diacétique de formule générale I 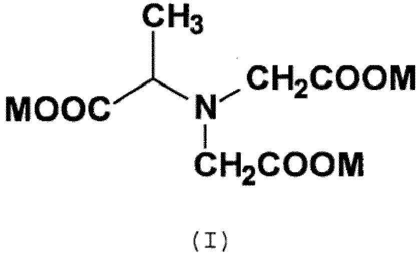
dans laquelle chaque M signifie indépendamment les uns des autres hydrogène, métal alcalin, ammonium ou ammonium substitué en les quantités stœchiométriques appropriées,
le solide conservant sa consistance de granulat lors d'un stockage ouvert à 25 °C et à une humidité relative de l'air de 76 % pendant une durée d'au moins un jour,
**caractérisé en ce qu'**au moins un composé cristallin de formule I est chargé et une granulation par pulvérisation est réalisée avec une solution aqueuse d'au moins un composé de formule I.

2. Procédé selon la revendication 1, **caractérisé en ce qu'**une partie du produit est soutirée du granulateur, broyée et introduite dans le granulateur en tant que nouveaux germes.

3. Procédé selon l'une quelconque des revendications 1 à 2, dans lequel le produit est post-traité thermiquement.
